(19) Europäisches Patentamt
European Patent Office
Office européen des brevets

(11) EP 0 826 667 B1

(12) **EUROPEAN PATENT SPECIFICATION**

(45) Date of publication and mention of the grant of the patent:
**30.10.2002 Bulletin 2002/44**

(51) Int Cl.[7]: **C07D 207/22**, C07C 233/51, A01N 43/36

(21) Application number: **97119172.1**

(22) Date of filing: **28.11.1991**

(54) **2 Aryl 5 (trifluoromethyl) 2 pyrroline compounds and process for the manufacture of insecticidal, 2 aryl 1 (alkoxymethyl) 4 halo 5 (trifluoromethyl) pyrroles**

2-Aryl-5-(trifluoromethyl)-2-pyrrolinderivate und Verfahren zur Herstellung von 2-Aryl-1-(alkoxymethyl)-4-halo-5-(trifluoromethyl)-pyrrolen mit insektizider Wirkung

Dérivés de 2-Aryl-5-(trifluorométhl)-2-pyrroline et procédé de préparation des 2-Aryl-1-(alkoxyméthyl)-4-halo-5-(trifluorométhyl)-pyrroles insecticides

(84) Designated Contracting States:
**AT BE CH DE DK ES FR GB GR IT LI LU NL SE**

(30) Priority: **26.12.1990 US 634288**
**26.12.1990 US 634287**

(43) Date of publication of application:
**04.03.1998 Bulletin 1998/10**

(62) Document number(s) of the earlier application(s) in accordance with Art. 76 EPC:
**91120369.3 / 0 492 171**

(73) Proprietor: **BASF AKTIENGESELLSCHAFT**
**67056 Ludwigshafen (DE)**

(72) Inventors:
- **Venkataraman, Kameswaran**
  **Mercer County, New Jersey 08550 (US)**
- **Doehner, Robert Francis Jr.,**
  **East Windsor, New Jersey 08520 (US)**
- **Barton, Jerry Michael,**
  **East Windsor, New Jersey 08520 (US)**
- **Kuhn, David Georg.**
  **Newton, Pennsylvania 18940 (US)**

(56) References cited:
**EP-A- 0 279 716**
**EP-A- 0 281 707**
**EP-A- 0 347 488**
**EP-A- 0 358 047**

- **J. P. CELERIER ET AL.: "Heterocyclization of primary amines with highly activated cyclopropanes: A new route isoretronecanol" TETRAHEDRON LETTERS, vol. 28, no. 52, 1987, GREAT BRITAIN, pages 6597-6600, XP002049891**

Remarks:
The file contains technical information submitted after the application was filed and not included in this specification

EP 0 826 667 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

It is an object of this invention to present more useful intermediates for the process of manufacture of a variety of insecticidal, acaricidal and nematocidal arylpyrrole compounds and a method for the preparation of said intermediates.

It is also an object of this invention to provide a process for the manufacture of 4-halo-2-aryl-1-(alkoxymethyl)-5-(trifluoromethyl)pyrrole compounds which are useful for controlling insect, acarid and nematode pests and for protecting harvested and growing crops from said pests.

It is another object of this invention to provide intermediate 4-halo-2-aryl-1-(halomethyl)-5-(trifluoromethyl) pyrrole compounds.

It is a further object of this invention to provide an insecticidal, acaricidal and nematocidal agent 4-chloro-1-(ethoxymethyl)-2-(p-chlorophenyl)-5-(trifluoromethyl)pyrrole-3-carbonitrile and a method for its use to protect harvested and growing crops.

EP-A-0 358 047 discloses a method for controlling phytopathogenic fungi using a cyano- or nitropyrrole compound.

EP-A-0 347 488 discloses arylpyrrole insecticidal, acaricidal and nematicidal agents and a method for the preparation thereof.

EP-A-0 281 707 discloses a process for the synthesis of β-phenylalanine.

EP-A-0 279 716 discloses a process for N-ω-trifluoroacetylation of saturated aliphatic α,ω-diamino carboxylic acids.

Tetrahedron Letters 28 (1987), 6597-6600 discloses synthesis of dihydropyrroles.

The present invention relates to compounds of formula I

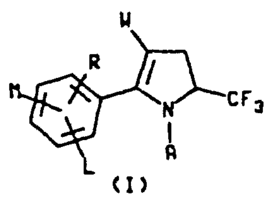

(I)

wherein

| | |
|---|---|
| A | is hydrogen or $C_1$-$C_4$ alkyl; |
| W | is CN, $NO_2$ or $CO_2R_6$; |
| L | is hydrogen or halogen and |
| M and R | are each independently hydrogen, $C_1$-$C_4$ alkyl, $C_1$-$C_4$ alkoxy, $C_1$-$C_4$ alkylthio, $C_1$-$C_4$ alkylsulfinyl, $C_1$-$C_4$ alkylsulfonyl, cyano, nitro, halogen, $CF_3$, $R_1CF_2Z$, $R_2CO$ or $NR_3R_4$ and when M and R are on adjacent positions they may be taken together with the carbon atoms to which they are attached to form a ring in which MR represents the structure |

$$-OCH_2O-, -OCF_2O- \text{ or } -CH=CH-CH=CH-;$$

| | |
|---|---|
| Z | is $S(O)_n$ or O; |
| $R_1$ | is hydrogen, F, $CHF_2$, CHFCl or $CF_3$ ; |
| $R_2$ | is $C_1$-$C_4$ alkyl, $C_1$-$C_4$ alkoxy or $NR_3R_4$; |
| $R_3$ | is hydrogen or $C_1$-$C_4$ alkyl; |
| $R_4$ | is hydrogen, $C_1$-$C_4$ alkyl or $R_5CO$; |
| $R_5$ | is hydrogen or $C_1$-$C_4$ alkyl and |
| $R_6$ | is $C_1$-$C_6$ alkyl, $C_3$-$C_6$ cycloalkyl or phenyl; |
| n | is an integer of 0, 1 or 2. |

This invention also relates to a method for the preparation of the above compounds and their use in the manufacture of arylpyrrole insecticidal agents.

The present invention also involves process for the preparation of compounds of formula II

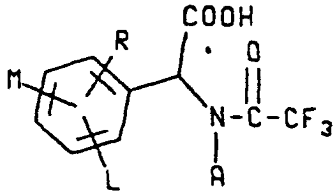

(II)

wherein A, L, M and R are as described above for formula I with the proviso that when A is hydrogen, then at least one of L, M and R must be other than hydrogen.

In addition, this invention is directed to a process for the manufacture of insecticidal arylpyrrole compounds of formula IV

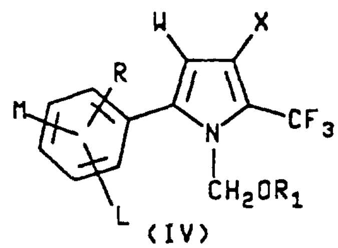

(IV)

wherein

| | |
|---|---|
| $R_1$ | is $C_1$-$C_6$ alkyl; |
| W | is CN, $NO_2$ or $CO_2R_2$; |
| X | is Br, Cl or I; |
| L | is hydrogen or halogen; |
| M and R | are each independently hydrogen, $C_1$-$C_4$ alkyl, $C_1$-$C_4$ alkoxy, $C_1$-$C_4$ alkylthio, $C_1$-$C_4$ alkylsulfinyl, $C_1$-$C_4$ alkylsulfonyl, CN, $NO_2$, Cl, Br, F, I, $CF_3$, $R_3CF_2Z$, $R_4CO$ or $NR_5R_6$ and when M and R are on adjacent positions they may be taken together with the carbon atoms to which they are attached to form a ring in which MR represents the structure |

$$-OCH_2O-, -OCF_2O- \text{ or } -CH{=}CH{-}CH{=}CH-;$$

| | |
|---|---|
| $R_2$ | is $C_1$-$C_6$ alkyl, $C_3$-$C_6$ cycloalkyl or phenyl; |
| $R_3$ | is hydrogen, F, $CHF_2$, CHFCl or $CF_3$; |
| $R_4$ | is $C_1$-$C_4$ alkyl, $C_1$-$C_4$ alkoxy or $NR_5R_6$; |
| $R_5$ | is hydrogen or $C_1$-$C_4$ alkyl; |
| $R_6$ | is hydrogen, $C_1$-$C_4$ alkyl or $R_7CO$; |
| $R_7$ | is hydrogen or $C_1$-$C_4$ alkyl; |
| Z | is $S(O)_n$ or O and |
| n | is an integer of 0, 1 or 2 which comprises reacting a compound of formula V |

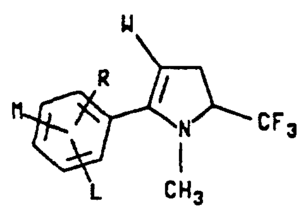

(V)

wherein W, L, M and R are as described hereinabove with at least 1 molar equivalent of a halogen, $X_2$, wherein $X_2$ is Br, Cl or I, preferably at an elevated temperature in the presence of a solvent to obtain a 2-aryl-1-methylpyrrole intermediate, reacting said 1-methylpyrrole intermediate with at least one additional molar equivalent of said halogen to form a 2-aryl-4-halo-1-methylpyrrole intermediate, reacting said 4-halo-1-methylpyrrole intermediate further with at least one molar equivalent of said halogen in the presence of a radical initiator to form a 2-aryl-4-halo-1-(halomethyl) pyrrole intermediate and reacting said 4-halo-1-(halomethyl)pyrrole intermediate with at least one molar equivalent of an alkali metal $C_1$-$C_6$alkoxide to give the compound of formula IV.

Compounds of formula I may be prepared by reacting compounds of formula II with about 1.0 molar equivalent of an activated olefin of formula III

$$H_2C{=}CHW$$

(III)

wherein W is CN, $NO_2$ or $CO_2R_6$ and $R_6$ is $C_1$-$C_6$ alkyl, $C_3$-$C_6$ cycloalkyl or phenyl in the presence of an acid anhydride and a solvent, optionally in the presence of an organic base. The method of preparation is illustrated in Flow Diagram I.

FLOW DIAGRAM I

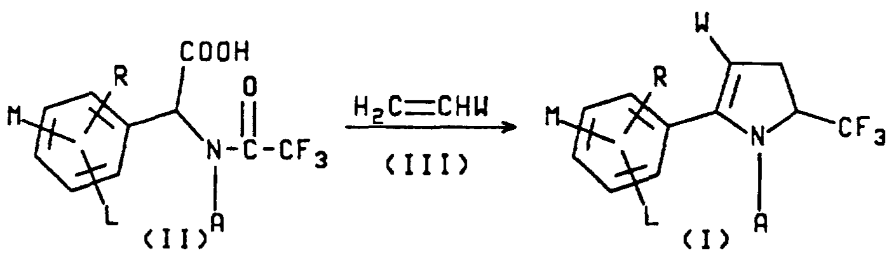


Solvents that may be used in the method of invention include aprotic organic solvents for example nitriles such as acetonitrile; esters such as ethyl acetate and methyl propionate; ethers such as diethyl ether, tetrahydrofuran, dioxane and ethylene glycol dimethyl ether; halogenated hydrocarbons such as methylene chloride, 1,2-dichloroethane, chloroform, 1,1,1-trichloroethane and carbon tetrachloride; carboxylic acid amides such as N,N-dimethylformamide and N-methylpyrrolidinone; sulfoxides such as dimethyl sulfoxide; sulfones such as tetramethylene sulfone; aromatic hydrocarbons such as benzene, toluene and xylene; halogenated aromatic hydrocarbons such as chlorobenzene and o-dichlorobenzene. One of the preferred organic solvents is acetonitrile. Acid anhydrides suitable for use in the method of invention are loweralkyl anhydrides such as acetic anhydride. Among the organic bases that may be used in the inventive method are pyridine, morpholine, tri($C_1$-$C_4$)alkylamine, hexamethylenetetramine and dimethylamino pyridine. A preferred organic base is a tri($C_1$-$C_4$)alkylamine such as triethylamine.

Compounds of formula II may be prepared from the appropriate arylaldehyde and suitable amine precursor via a Strecker synthesis to form the amino acid intermediate of formula III which may be trifluoroacetylated to obtain the desired formula II compound as shown in Flow Diagram II.

## FLOW DIAGRAM II

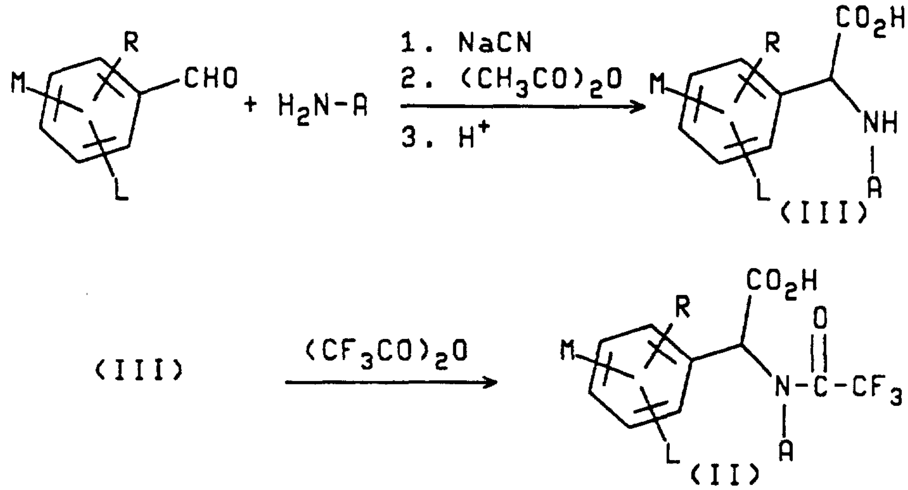


Compounds of formula II wherein A is hydrogen may also be prepared via trifluoroacetylation of the appropriate arylglycine precursor. Alkylation of the thus-obtained compound using an alkylating agent such as a lower alkylhalide gives compounds of formula II wherein A is $C_1$-$C_4$ alkyl. Using methyl iodide as the alkylating agent, the reaction sequence is illustrated in flow diagram III.

## FLOW DIAGRAM III

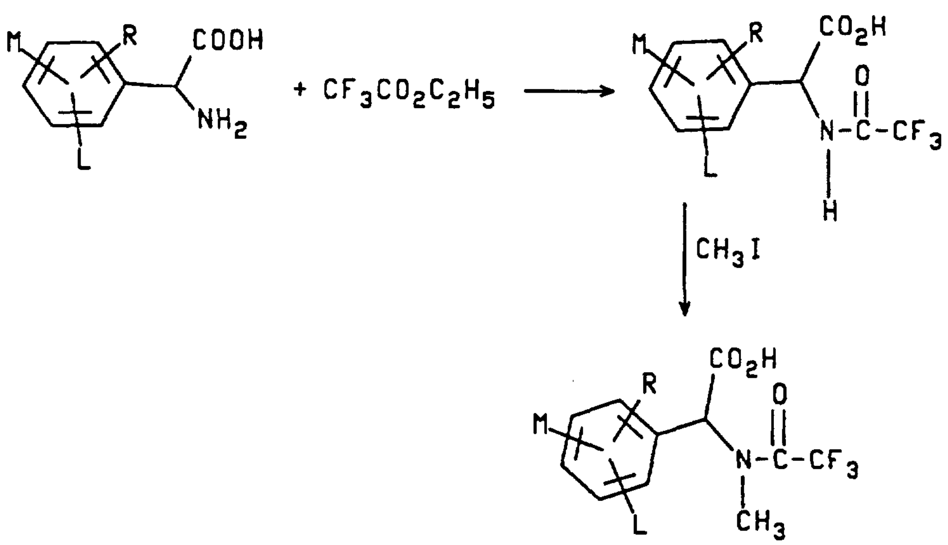


The compounds of the invention are intermediates in the manufacture of a variety of arylpyrroles useful as insecticidal, nematocidal and acaricidal agents. Among the arylpyrrole insecticidal agents that may be prepared from the compounds of the invention are 4-halo-2-(p-chlorophenyl)-1-(substituted)-5-(trifluoromethyl)pyrrole-3-carbonitrile compounds. In one example of the method of the invention, the formula I pyrroline compound wherein A is methyl, W is CN, L and R are hydrogen and M is Cl may be reacted with a halogen in the presence of an aprotic solvent at an elevated temperature to give insecticidal arylpyrrole agents of formulas IV and V wherein X is chlorine, bromine or iodine. Further reaction with additional halogen in the presence of a radical initiator such as benzoyl peroxide, 2,2'-azobisisobutyronitrile, photochemical irradiation and the like yields the corresponding 1-(halomethyl)pyrrole intermediate of formula VI which can be reacted with an alkali metal alkoxide such as sodium ethoxide, potassium methoxide or the like to give an arylpyrrole insecticidal agent of formula VII. The reactions are shown in Flow Diagram IV.

FLOW DIAGRAM IV

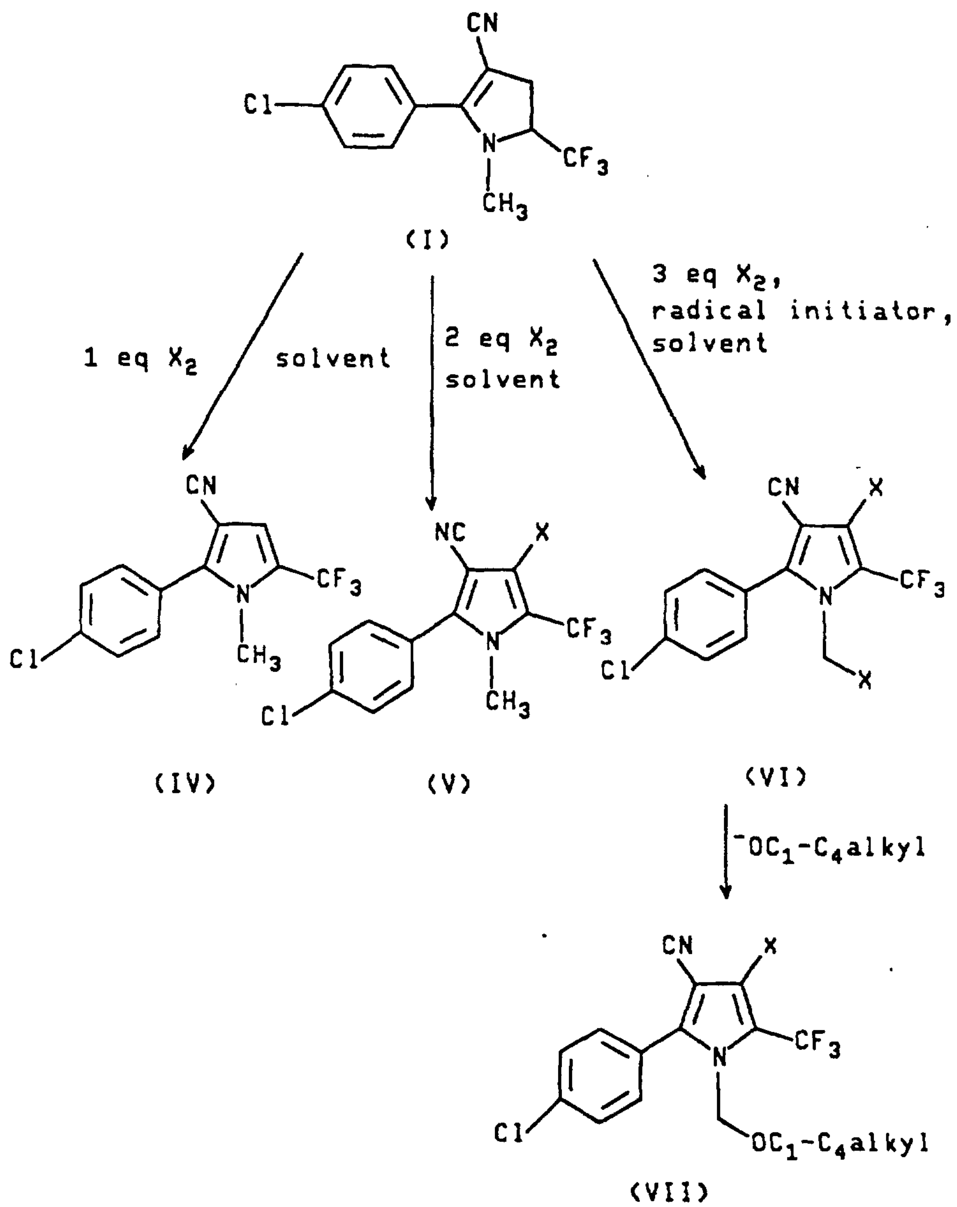


(I) (IV) (V) (VI) (VII)

By varying the substituents A, W, L, M and R, the halogen reactant and the reaction conditions, a wide variety of arylpyrrole insecticidal, nematocidal and acaricidal agents may be manufactured from the compounds of formula I.

The compounds of formula V are particularly effective for controlling insect, acarid and nematode pests and for protecting agronomic crops, both growing and harvested, against the ravages of these pests. Methods of preparation for the compounds generally include halogenation of 2-aryl-5-(trifluoromethyl)-pyrrole-3-carbonitrile with a halogenating agent such as a hypohalite, a halogen or a sulfurylhalide to form the corresponding 4-halopyrrole intermediate and alkylating said intermediate with an appropriate chloromethyl($C_1$-$C_6$ alkyl)ether.

It has now been found that compounds of formula I, particularly wherein X is chlorine, may be readily and efficiently manufactured from pyrroline compounds of formula II by the incremental addition of the appropriate halogen in the presence of a solvent preferably at an elevated temperature to form a 4-halo-1-methylpyrrole intermediate of formula III, the in situ reaction of formula III intermediate with additional halogen in the presence of a radical initiator

to form a 4-halo-1-(halomethyl)pyrrole intermediate of formula IV and the reaction of formula IV intermediate with an alkali metal $C_1$-$C_6$alkoxide. Surprisingly, the desired compounds of formula I are obtained in good yields and have a high degree of purity. The process of the present invention is shown in flow diagram I.

FLOW DIAGRAM I

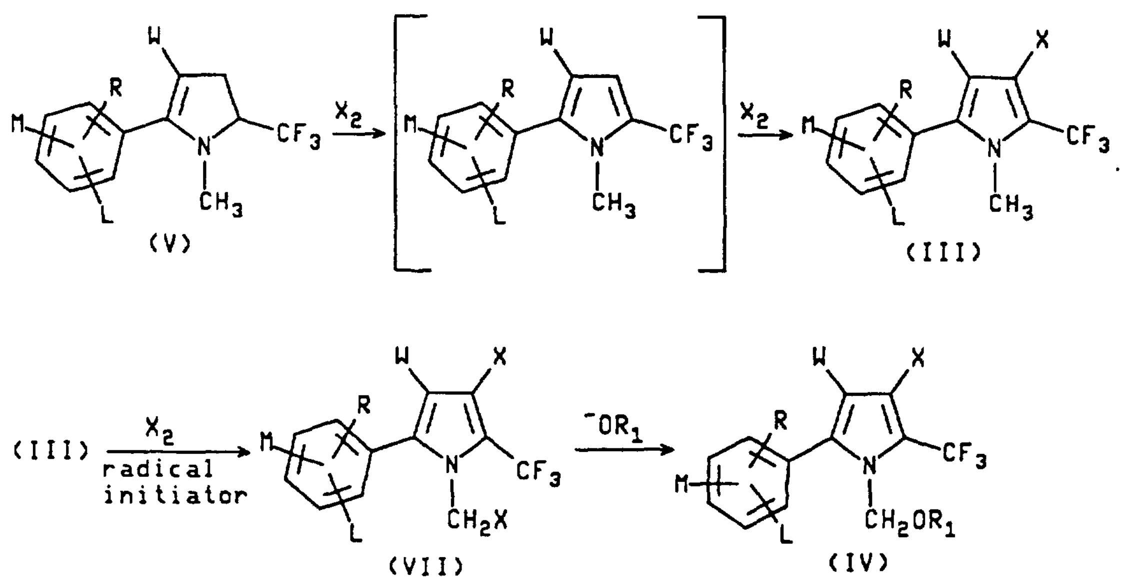


Advantageously, it has been found that the incremental addition of halogen improves the reaction yield and product purity. The process of the invention also improves the environmental and human safety of the manufacture of compounds of formula I by avoiding the use of certain undesirable reactants such as chloromethylethylether. Further, the process of the invention includes the productive manufacture of 2-(p-chlorophenyl)-4-chloro-1-ethoxymethyl-5-(trifluoromethyl)pyrrole-3-carbonitrile, an effective insecticidal, acaricidal and nematocidal agent.

Solvents suitable for use in the process of the present invention include aprotic solvents, for example halogenated aromatic hydrocarbons such as chlorobenzene and o-dichlorobenzene and halogenated hydrocarbons such as methylene chloride, carbon tetrachloride, chloroform and 1,2-dichloroethane. Preferred solvents include halogenated aromatic hydrocarbons such as chlorobenzene and halogenated hydrocarbons such as carbon tetrachloride. Radical initiators suitable for use are light, peroxides such as dibenzoyl peroxide, di-t-butylperoxide, and the like; azo compounds such as azobisisobutyronitrile. Among the preferred radical initiators are benzoyl peroxide and light. Alkali metal alkoxides suitable for use in the inventive method are sodium ethoxide and potassium methoxide.

In the process of the invention the reaction rate is increased with increased temperature so that elevated temperatures in the range of 55° to 210°C, preferably 70° to 120°C, allow the oxidation, ring halogenation and side chain halogenation to proceed at an efficient and effective rate without causing undue adverse side reactions.

It is now apparent that the process of the invention includes the operative manufacture of an insecticidal, acaricidal and nematocidal agent, 2-(p-chlorophenyl)-4-chloro-1-(ethoxymethyl)-5-(trifluoromethyl)pyrrole-3-carbonitrile. The pyrrole-3-carbonitrile is e.g. effective for controlling acarina, lepodoptera, colioptera, hemiptera. Among the agronomic pests that may be effectively controlled are potato beetle, cabbage looper, diamond back moth, tobacco budworm, corn earworm, beet armyworm and tobacco bollworm. Crops which may be protected by the compound of the invention are lettuce, broccoli, corn, cabbage, cauliflower, tomato and cotton.

In practice, generally 10 ppm to 10,000 ppm, preferably 100 to 5,000 ppm of 2-(p-chlorophenyl)-4-chloro-1-(ethoxymethyl)-5-(trifluoromethyl)-pyrrole-3-carbonitrile dispersed in water or other suitable liquid carrier, is effective when applied to the crops or the soil in which the crops are growing to protect the crops from attack by insects, acarina and nematodes. The compound is also effective for protecting horticultural plants such as turf grass from attack by pests such as grubs, chinch bugs and the like.

While the 4-chloro-1-(ethoxymethyl)pyrrole compound of the invention is effective for controlling insects, acarina and nematodes when employed alone, it may also be used in combination with other pesticidal compounds including other nematocides and acaricides. For example, the compound of this invention may be employed effectively

in combination with other arylpyrroles, phosphates, carbamates, pyrethroids, formamidines, chlorinated hydrocarbons and halobenzoylureas. .

In order to facilitate a further understanding of the invention, the following examples are presented to illustrate more specific details thereof. The term NMR designates nuclear magnetic resonance and the term HPLC designates high pressure liquid chromatography. Unless otherwise noted, all parts are parts by weight.

## EXAMPLE 1

### Preparation of 2-(p-Chlorophenyl)sarcosine

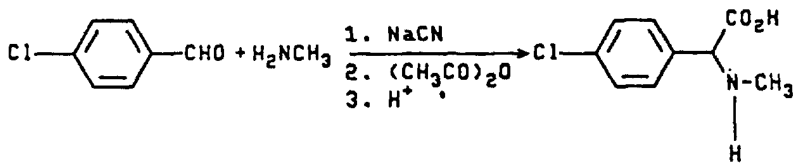


A mixture of p-chlorobenzaldehyde (153 g, 1.0 mol) in tetrahydrofuran is treated with a solution of methylamine hydrochloride (88 g, 1.3 mol) in water followed by an aqueous solution of sodium cyanide (53 g, 1.0 mol), stirred at room temperature for 16 hours and extracted with toluene. The organic extract is treated with 10 cc of pyridine followed by 50 cc of acetic anhydride (exotherm), stirred at ambient temperatures for 1/2 hour and concentrated in vacuo to give an oil residue. The residue is added to a 1:1 mixture of water and concentrated hydrochloric acid, heated at reflux temperature for 2 hours, cooled, diluted with water and neutralized to about pH 2 with 50% NaOH solution. The resultant solid precipitate is filtered and air-dried to give the title product as a white solid, 180 g (89.5% yield), mp 208-213°C.

## EXAMPLE 2

### Preparation of 2-(p-Chlorophenyl)-N-(trifluoroacetyl)-sarcosine

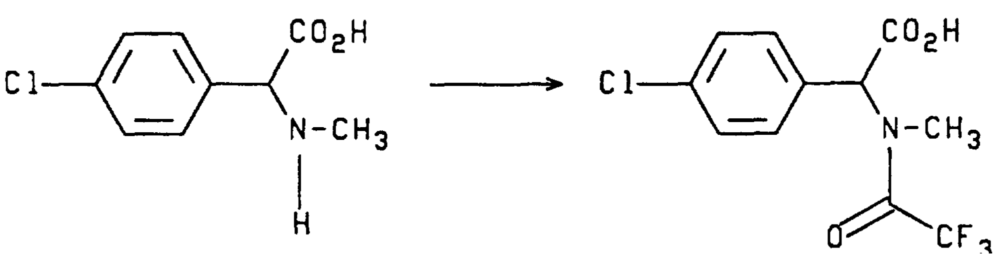

A mixture of 2-(p-chlorophenyl)sarcosine (27 g, 0.135 mol) in dry toluene is treated with 20 cc of trifluoroacetic anhydride, stirred for 1 hour and concentrated in vacuo to give a solid residue. The residue is re-evaporated several times with toluene to give the title product as a red solid, 38.7 g (97% yield), mp 117-118°C, identified by NMR spectroscopy.

**EXAMPLE 3** (reference example) **Preparation of 2-phenyl-N-(trifluoroacetyl)glycine**

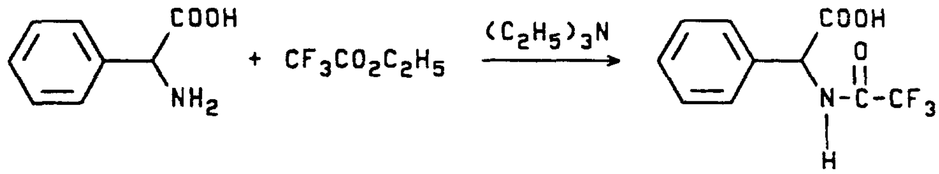


A mixture of DL-phenylglycine (15.1 g, 0.10 mol) in methanol is treated with triethylamine (10.1 g, 0.10 mol) and ethyl trifluoroacetate (17.8 g, 0.125 mol), stirred at room temperature for 72 hours, diluted with methanol and treated with Dowex 50x8 acidic resin. The reaction mixture is stirred for 10 minutes and filtered. The filtrate is concentrated in vacuo to give a pale yellow solid which is recrystallized from 1,2-dichloroethane to give the title product as white needles, 10.5 g (42% yield), mp 155-157°C.

**EXAMPLE 4**

**Preparation of 2 -p-chlorophenyl-N-(trifluoroacetyl)-glycine**

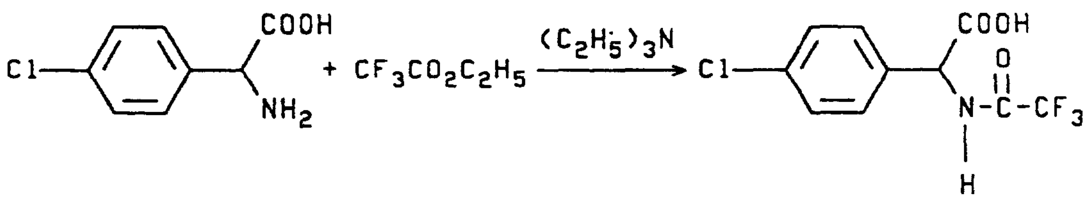


A mixture of 2-(p-chlorophenyl)glycine (37.1 g, 0.2 mol) in methanol is treated with triethylamine (20.2 g, 0.2 mol), stirred for 10 minutes, treated dropwise with ethyl trifluoroacetate (35.5 g, 0.25 mol), stirred for 4 days, diluted with methanol and treated with Dowex 50x8-100 ion exchange resin. The reaction mixture is stirred for 10 minutes and filtered. The filter cake is washed with methanol. The filtrates are combined and concentrated in vacuo to afford a yellow solid which is recrystallized from 1,2-dichloroethane to give the title compound as white crystals, 26.4 g (46.8% yield), mp 170-172°C.

**EXAMPLE 5** (reference example) **Preparation of 2-Phenyl-N-(trifluoroacetyl)sarcosine**

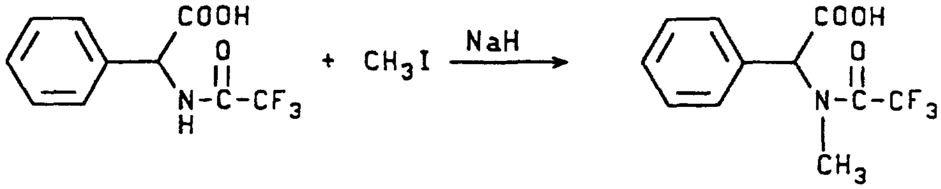


A mixture of 2-phenyl-N-(trifluoroacetyl)-glycine (2.5 g, 0.01 mol) and iodomethane (11.35 g, 0.08 mol) in tetrahydrofuran is treated portionwise with a 60% dispersion of sodium hydride in mineral oil (1.2 g, 0.03 mol NaH),

stirred at ambient temperatures for 1 hour, heated at reflux temperature for 17 hours, cooled to room temperature, diluted with ethyl acetate followed by 1 mL of water and concentrated in vacuo to a wet yellow solid residue. The residue is dispersed in a mixture of ether and water. The ether layer is washed with a sodium bicarbonate solution. The aqueous phases are combined, acidified with 10% HCl and extracted with ethyl acetate. The ethyl acetate extract is washed sequentially with water, sodium thiosulfate and saturated sodium chloride solution, dried over $MgSO_4$ and concentrated in vacuo to give a pale yellow solid which is recrystallized from methylcyclohexane to give the title compound as white crystals, 0.5 g, (19.1% yield), mp 124-126°C.

**EXAMPLE 6**

**Preparation of 2-(p-Chlorophenyl)-1-methyl-5-(trifluoromethyl)-2-pyrroline-3-carbonitrile**

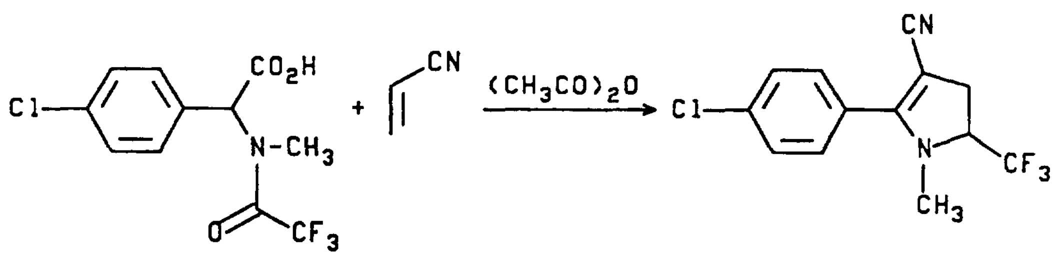


A solution of 2-(p-chlorophenyl)-N-(trifluoromethyl)sarcosine (7.4 g, 0.02 mol) in acetonitrile is treated with acetic anhydride (5.1 g, 0.05 mol), acrylonitrile (1.6 g, 0.03 mol) and 10 drops of triethylamine, heated at reflux temperature for 5 1/2 hours, cooled and concentrated in vacuo to a red oil residue. The residue is filtered through silicagel using 9:1 hexanes/ethyl acetate followed by mixtures of methylene chloride and ethyl acetate. The combined filtrates are concentrated in vacuo to give the title product as a red solid, 6.1 g (85% yield), identified by NMR and mass spectral analyses. A portion of the solid is recrystallized from methylene chloride to give light yellow needles, mp 158-160°C.

**EXAMPLE 7**

**Preparation of 2 - (p-Chlorophenyl)-5 -(trifluoromethyl)-2-pyrroline-3-carbonitrile**

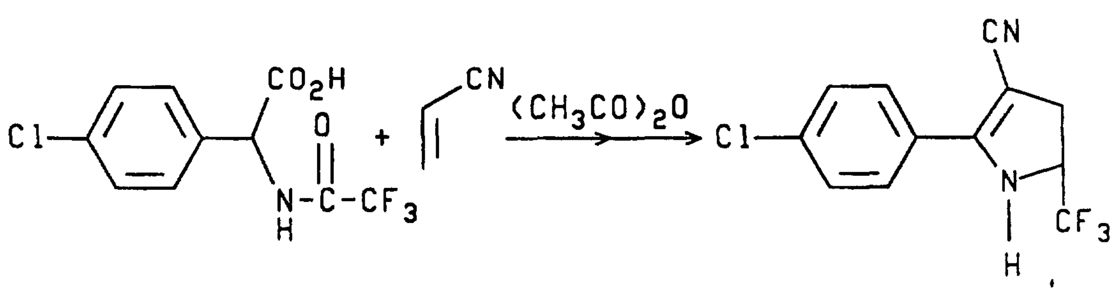


Using essentially the same procedure described in Example 6 and substituting 2-(p-chlorophenyl)-N-(trifluoroacetyl)glycine as starting material affords the title product as a light yellow solid, mp 158-160°C.

**EXAMPLE 8**

**Preparation of 4-Chloro-1-chloroaethyl-2-(p-chlorophenyl)-5-(trifluoromethyl)pyrrole-3-carbonitrile**

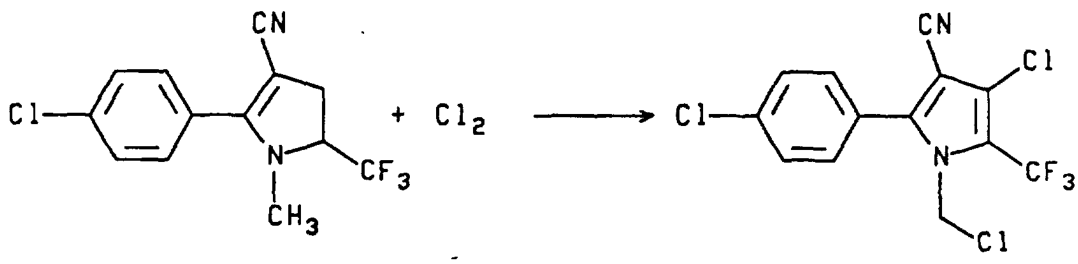

To a solution of 2-(p-chlorophenyl)-1-methyl-5-(trifluoromethyl)-2-pyrroline-3-carbonitrile (2.85 g, 0.01 mol) in o-dichlorobenzene is added chlorine (0.8 g, 0.011 mol); the resulting solution is stirred at room temperature for 1 hour, heated slowly to 90°C over a 2 hour period, cooled to 50°C, treated with additional chlorine (0.8 g, 0.11 mol), heated at 110°C for 24 hours, cooled to room temperature; treated with additional chlorine and concomitant additions of small portions of benzoyl peroxide and'heated at 110°C for a 4 hour period or until reaction is complete by chromatographic analysis. The resultant solution is cooled, washed with sodium metabisulfite solution and concentrated in vacuo to give a residue which is crystallized by the addition of heptane to afford the title product as a white solid, mp 107-108°C.

**EXAMPLE 9**

**Preparation of 4-Chloro-2-(p-chlorophenyl)-1-ethoxymethyl)-5-trifluoromethyl)pyrrole-3-carbonitrile**

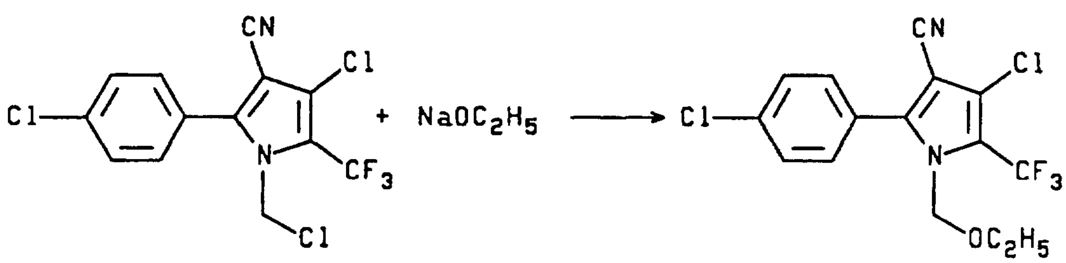

A solution of 4-chloro-1-(chloromethyl)-2-(p-chlorophenyl)-5-(trifluoromethyl)pyrrole-3-carbonitrile (2.6 g, 0.0074 mol) in tetrahydrofuran is treated with sodium ethoxide as a 21% wt/wt solution in denatured ethanol (3.6 mL, 0.0096 mol), stirred at room temperature for 1 hour, treated with an additional 2-3 drops of the sodium ethoxide solution, heated at reflux temperature for 1 hour, cooled and poured into water. The resultant precipitate is filtered, dried and recrystallized from isopropanol to afford the title product as a white solid, 1.6 g (60% yield), mp 104.0-104.5°C.

**EXAMPLE 10**

**Preparation of 2-(p)-Chlorophenyl)-1-methyl-5-(trifluoromethyl)pyrrole-3-carbonitrile**

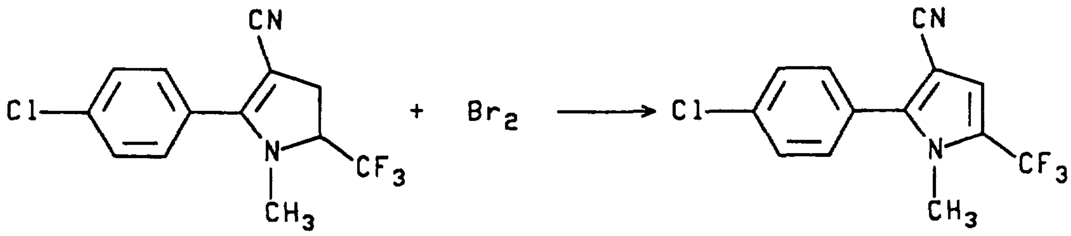

A mixture of 2-(p-chlorophenyl)-1-methyl-5-(trifluoromethyl)-2-pyrroline-3-carbonitrile (2.87 g, 0.01 mol) in chlorobenzene is treated with a solution of bromine (1.76 g, 0.011 mol) in chlorobenzene and heated at 100°C for 4-5 hours (until the reaction is complete by HPLC analysis). The reaction mixture is cooled to room temperature, diluted with water and extracted with ethyl acetate. The organic extracts are combined, washed sequentially with water, sodium metabisulfite and water, dried over $MgSO_4$ and concentrated in vacuo to give a pale yellow solid residue. The solid is recrystallized from heptane/ethyl acetate to give the title product as pale yellow crystals, 2.4 g, (84.2% yield), mp 129.5-130°C.

**EXAMPLE 11**

**Preparation of 4-Bromo-2-(p)-Chlorophenyl)-1-methyl-5-(trifluoromethyl)pyrrole-3-carbonitrile**

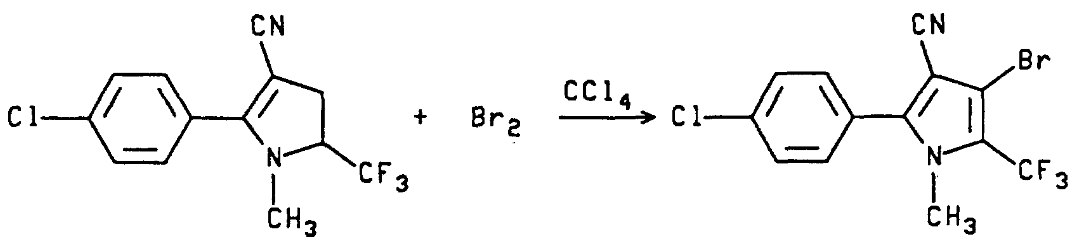


A mixture of 2-(p-chlorophenyl)-1-methyl-5-(trifluoromethyl)-2-pyrroline-3-carbonitrile (2.87 g, 0.01 mol) in carbon tetrachloride and bromine (3.2 g, 0.015 mol) is heated at reflux temperature for 2 hours, cooled to room temperature, treated with additional bromine (3.2 g, 0.015 mol), heated at reflux temperature for 6-7 hours (until reaction is complete by HPLC analysis), cooled to room temperature, washed with aqueous sodium metabisulfite and concentrated in vacuo to give a residue. The residue is recrystallized from heptane to give the title product as white crystals, mp 131-131.5°C.

**EXAMPLE 12**

**Preparation of 4-Chloro-1-(chloromethyl)-2-(p-chlorophenyl)-5-(trifluoromethyl)pyrrole-3-carbonitrile**

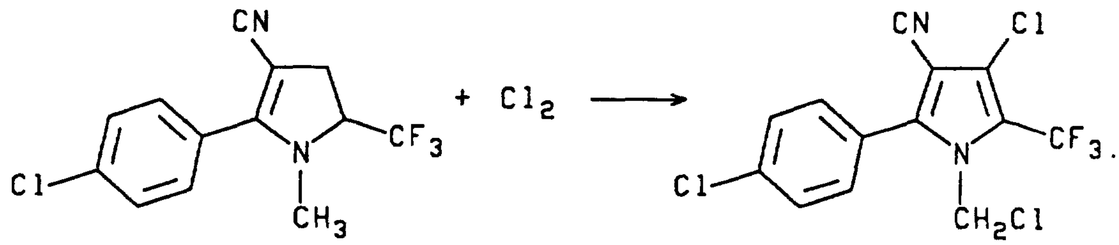

To a solution of 2-(p-chlorophenyl)-1-methyl-5-(trifluoromethyl)-2-pyrroline-3-carbonitrile (2.85 g, 0.01 mol) in chlorobenzene is added chlorine (0.8 g, 0.011 mol), stirred at room temperature for 1 hour, heated at 90°C for 2 hours, cooled to room temperature, treated with additional chlorine (1.1 g, 0.015 mol), heated at 110°C for 24 hours, cooled to room temperature, treated further with additional chlorine (1.4 g, 0.02 mol) and a catalytic amount of benzoyl peroxide and heated at 110°C until reaction is complete by HPLC analysis. The mixture is cooled to room temperature, washed with aqueous sodium metabisulfite, dried ($MgSO_4$) and concentrated in vacuo to give a residue. The residue is recrystallized from heptane to give the title product as a white crystalline solid, mp 107-108°C.

**EXAMPLE 13**

**Preparation of 4-Bromo-1-(bromomethyl)-2-(p -chlorophenyl)-5-(trifluoromethyl)pyrrole-3-carbonitrile**

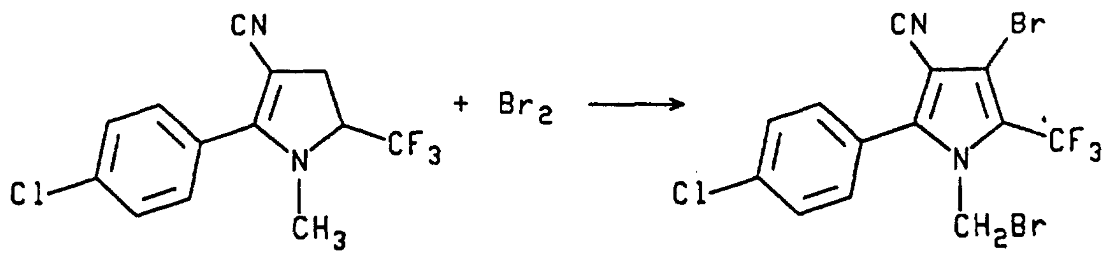

A stirred solution of 2-(p-chlorophenyl)-1-methyl-5-(trifluoromethyl)-2-pyrroline-3-carbonitrile (2.85 g, 0.01 mol) in carbon tetrachloride is treated with 1.1 molar equivalents of bromine at room temperature, heated at 70°C for 2 hours, cooled to room temperature, treated with an additional 1.5 molar equivalents of bromine, heated at reflux temperature for 6-12 hours, cooled to room temperature, treated with a further 1.5 equivalents of bromine, irradiated for 2-4 days at 70°C or until reaction is complete by HPLC analysis, cooled to room temperature and washed with aqueous sodium meta bisulfite. The organic phase is concentrated in vacuo to give a residue which is recrystallized from heptane to give the title product as white crystals, mp 131.0-131.5°C.

**EXAMPLE 14**

**Preparation of 4-Chloro-2-(p-chlorophenyl)-1-(ethoxymethyl)-5-(trifluoronethyl)pyrrole-3-carbonitrile**

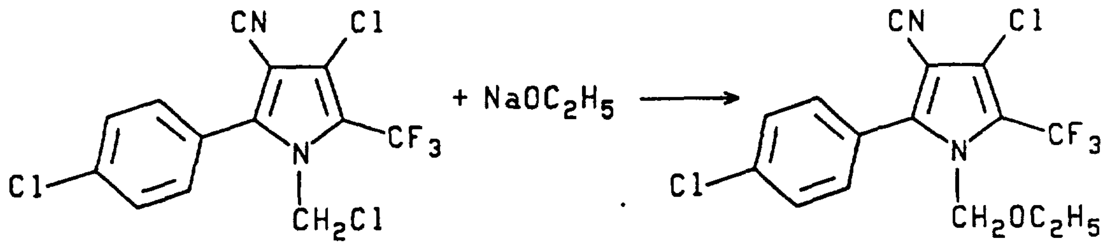


A solution of 4-chloro-1-(chloromethyl)-2-(p-chlorophenyl)-5-(trifluoromethyl)pyrrole-3-carbonitrile (2.6 g, 0.0074 mol) in tetrahydrofuran is treated with sodium ethoxide as a 21% wt/wt solution in denatured ethanol (3.6 mL, 0.0096 mol), stirred at room temperature for 1 hour, treated with an additional 2-3 drops of the sodium ethoxide solution, heated at reflux temperature for 1 hour, cooled and poured in water. The resultant precipitate is filtered, dried and recrystallized from isopropanol to afford the title product as a white solid, 1.6 g (60% yield), mp 104.0-104.5°C.

**EXAMPLE 15**

**Preparation of 4-Bromo-1-(ethoxymethyl)-2-(p-chlorophenyl)-5-(trifluoromethyl)pyrrole-3-carbonitrile**

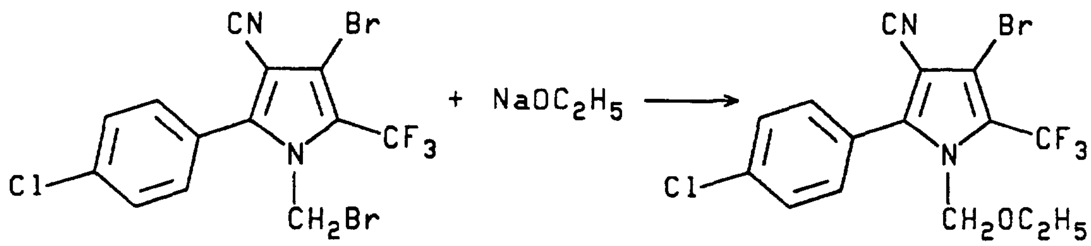


A mixture of 4-brono-1-(bromomethyl)-2-(p-chlorophenyl)-5-(trifluoromethyl)pyrrole-3-carbonitrile (4.42 g, 0.01 mol) in absolute ethanol is treated with a 21% wt/wt ethanolic solution of sodium ethoxide (0.715 g, 0.011 mol), heated to 80°C for 15-20 minutes, cooled to room temperature and diluted with water and ether. The organic phase is dried ($MgSO_4$) and concentrated in vacuo to give a solid residue which is recrystallized from heptane to give the title product as a white solid, 3.45 g (85% yield), mp 91-92°C.

**EXAMPLE 16**

**Insecticide and Acaricide Evaluation**

All tests are performed using technical material. All concentrations reported herein are in terms of active ingredient. All tests are kept at 27°C.

**Spodoptera eridania, 3rd instar larvae, southern armyworm**

A Sieva lima bean leaf expanded to 7-8 cm in length is dipped in the test suspension with agitation for 3 seconds and placed in a hood to dry. The leaf is then placed in a 100x10 mm petri dish containing a damp filter paper on the bottom and ten 3rd instar caterpillars. The dish is maintained for 5 days before observations are made of mortality, reduced feeding, or any interference with normal moulting.

**Spodoptera eridania, 7 day residual**

The plants treated in the above test are maintained under high intensity lamps in the greenhouse for 7 days. These lamps duplicate the effects of a bright sunny day in June in New Jersey and are kept on for 14 hour day length. After 7 days, the foliage is sampled and evaluated as described above.

**Tetranychus urticae(P-resistant strain),2-spotted spider mite**

Sieva lima bean plants with primary leaves expanded to 7-8 cm are selected, cut back to one plant per pot and infested with mites to obtain about 100 mites per leaf. The mite-infested plants are dipped in the test formulation for 3 seconds with agitation and set in the hood to dry. Plants are kept for 2 days before estimates of adult kill are made using the first leaf. The second leaf is kept on the plant for another 5 days before observations are made of the kill of eggs and/or newly emerged nymphs.

**Diabrotic undecimpunctata howardi, 3rd instar southern corn rootworm**

One cc of fine talc is placed in a 30 mL wide-mouth screw-top glass jar. One mL of the appropriate acetone suspension is pipetted onto the talc so as to provide 1.25 and 0.25 mg of active ingredient per jar. The jars are set under a gentle air flow until the acetone is evaporated. The dried talc is loosened, 1 cc of millet seed is added to serve as food for the insects and 25 mL of moist soil is added to each jar. The jar is capped and the contents thoroughly mixed on a Vortex Mixer. Following this, ten 3rd instar root-worms are added to each jar and the jars are loosely capped to allow air exchange for the larvae. The treatments are held for 6 days before mortality counts are made. Missing larvae are presumed dead, since they decompose rapidly and can not be found. The concentrations used in this test correspond approximately to 50 and 10 kg/ha, respectively.

**Aphis fabae, mixed instar, bean aphid**

Pots containing single nasturtium plants (Tropaeolum sp) about 5 cm tall are infested with about 100-200 aphids one day before the test. Each pot is sprayed with the test formulation for 2 revolutions of a 4 rpm turntable in a hood, using a #154 DeVilbiss atomizer. The spray tip is held about 15 cm from the plant and the spray directed so as to give complete coverage of the plants and the aphids. The sprayed pots are set on their sides on white enamel trays and held for 2 days, following which mortality estimates are made.

**Empoasca abrupta, adults, western potato leafhopper**

A Sieva lima bean leaf about 5 cm long is dipped in the test formulation for 3 seconds with agitation and placed in a hood to dry. The leaf is placed in a 100x10 mm petri dish containing a moist filter paper on the bottom. About 10 adjult leafhoppers are added to each dish and the treatments are kept for 3 days before mortality counts are made.

**Heliothis virescens, 3rd instar tobacco budworm**

Cotton cotyledons are dipped in the test formulation and allowed to dry in a hood. When dry, each is cut into quarters and ten sections placed individually in 30 mL plastic medicine cups containing a 5-7 mm long piece of damp dental wick. One third-instar caterpillar is added to each cup and a cardboard lid placed on the cup. Treatments are maintained for 3 days before mortality counts and estimates of reduction in feeding damage are made.

**Heliothis virescens, egg, tobacco budworm**

A young cotton leaf about 6-7 cm long is dipped in the test suspension with agitation for 3 seconds. Eggs are collected on cheesecloth which is cut into 10-20 mm squares containg about 50-100 eggs (6-30 hours old). A square of cheesecloth with eggs is also dipped in the test suspension and placed on the treated leaf. The combination is dried and placed in a cup (240 mL, 6 cm tall, top diameter 9.5 cm, bottom diameter 8 cm), into which a 5 cm length of damp dental wick has been placed. A clear plastic lid is placed on the cup and the treatments are held for 3 days before mortality counts are made.

**Blattella germanica, bait test, adult male German cockroach**

A 0.1% bait is prepared by pipetting a 1 mL of a 1000 ppm solution of the test compound in acetone onto 1 gram of cornmeal in a 30 mL wide-moputh bottle., The bait is dried by passing a gentle stream of air into the bottle. The bait is placed in a 1 pint wide-mouth Mason jar and ten adult male cockroaches are added. A screen lid is placed on the jar and a small piece of cotton soaked in 10% honey is put on the top of the screen lid. Mortality counts are made after 3 days.

**Blattela germanica, residue test, adult male German cockroach**

One mL of a 1000 ppm acetone solution of the test material is pipetted slowly over the bottom of a 150 x 15 mm petri dish so as to give as uniform coverage as possible. After the deposit has dried, 10 adult male cockroaches are placed in each dish and the lid is added. Mortality counts are made after 3 days.

| | |
|---|---|
| 0 = no effect | 5 = 56 - 65% kill |
| 1 = 10 - 25% kill | 6 = 66 - 75% kill |
| 2 = 26 - 35% kill | 7 = 76 - 85% kill |
| 3 = 36 - 45% kill | 8 = 86 - 99% kill |
| 4 = 46 - 55% kill | 9 = 100% kill |
| | R - reduced feeding |

The data obtained for the above-described evaluations are reported in Table I.

**TABLE I**

**Insecticide and Acaricide Evaluation of 2-(p-chlorophenyl)-4-chloro-1-(ethoxymethyl)-5-(trifluoromethyl)pyrrole-3-carbonitrile**

| | Evaluation | | | | |
|---|---|---|---|---|---|
| Test | 1,000 ppm | 100 ppm | 10 ppm | 1 ppm | 0.1 ppm |
| Southern Armyworm | | | | | |
| (3rd instar larvae) | 9 | 9 | 9 | 0 | - |
| (7 day residual) | 9 | 9 | 6 | - | - |
| 2-Spotted Spider Mite | - | - | 9 | 0 | - |
| Bean Aphid | - | 9 | 4 | 0 | - |
| Leafhopper | - | 9 | 9 | 9 | - |
| Tobacco Budworm | | | | | |
| (Eggs) | 7 | 0 | - | - | - |
| (3rd instar) | 9 | 9 | 9 | - | - |
| German Cockroach | | | | | |
| (Bait) | 0 | - | - | - | - |
| (Residue) | 9 | 0 | - | - | - |

| | 50 kg/ha | 10 kg/ha | 1 kg/ha |
|---|---|---|---|
| Southern Corn Rootworm | 9 | 7 | 0 |

## Claims

1. A compound **characterized by** the structure I

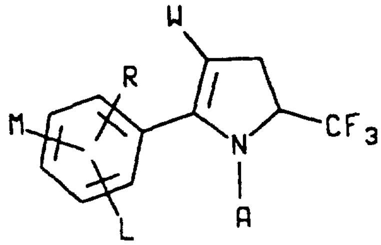

(I)

wherein

A is hydrogen or $C_1$-$C_4$ alkyl;
W is CN, $NO_2$ or $CO_2R_6$;
L is hydrogen or halogen and
M and R are each independently hydrogen, $C_1$-$C_4$ alkyl, $C_1$-$C_4$ alkoxy, $C_1$-$C_4$ alkylthio, $C_1$-$C_4$ alkylsulfinyl, $C_1$-$C_4$ alkylsulfonyl, CN, $NO_2$, halogen, $CF_3$, $R_1CF_2Z$, $R_2CO$ or $NR_3R_4$ and when M and R are on adjacent positions they may be taken together with the carbon atoms to which they are attached to form a ring in which MR represents the structure

$-OCH_2O-$, $-OCF_2O-$ or $-CH=CH-CH=CH-$;

Z is $S(O)_n$ or O;
$R_1$ is hydrogen, F, $CHF_2$, CHFCl or $CF_3$;
$R_2$ is $C_1$-$C_4$ alkyl, $C_1$-$C_4$ alkoxy or $NR_3R_4$;
$R_3$ is hydrogen or $C_1$-$C_4$ alkyl;
$R_4$ is hydrogen, $C_1$-$C_4$ alkyl or $R_5CO$;
$R_5$ is hydrogen or $C_1$-$C_4$ alkyl and
$R_6$ is $C_1$-$C_6$ alkyl, $C_3$-$C_6$ cycloalkyl or phenyl;
n is an integer of 0, 1 or 2.

**2.** The compound according to claim 1, selected from the group consisting of 2-(p-chlorophenyl)-1-methyl-5-trifluoromethyl)-2-pyrroline-3-carbonitrile and 2-(p-chlorophenyl)-5-(trifluoromethyl)-2-pyrroline-3-carbonitrile.

**3.** A process for the preparation of a compound according to claim 1, which comprises reacting a compound having structure II

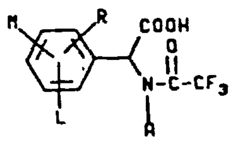

II

wherein A, L, M and R are as defined in claim 1 with about 1.0 molar equivalent of an olefin having the structure

$H_2C=CHW$,

wherein W is as defined above and optionally a catalytic amount of an organic base in the presence of an acid anhydride and a solvent.

**4.** A process according to claim 3 whereby the compound of structure II

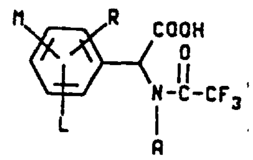

II

wherein A, L, M, and R are as defined in claim 1 with the proviso that when A is hydrogen, then at least one of L, M and R must be other than hydrogen, is prepared by reacting an aldehyde having the structure

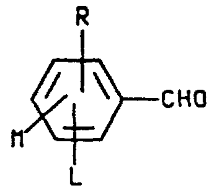

wherein L, M and R are as defined above with at least one molar equivalent of an amine hydrochloride having the structure

$$H_2N\text{-}A \cdot HCl$$

wherein A is as described above in the presence of a solvent to form an intermediate, reacting said intermediate solution with an aqueous solution containing at least one molar equivalent of an alkali metal cyanide to form an amino nitrile intermediate, extracting said amino nitrile intermediate from the reaction mixture with a suitable solvent, reacting the amino nitrile intermediate with an acid anhydride in the presence of an organic base to form an amido nitrile intermediate, reacting said amido nitrile with a mineral acid in the presence of water, optionally at an elevated temperature, to give an amino acid intermediate and reacting said intermediate with trifluoroacetic anhydride to give the desired compound having structure II.

**5.** A process for the manufacture of a compound **characterized by** formula IV

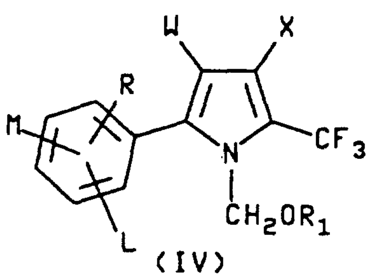

(IV)

wherein

$R_1$ is $C_1$-$C_6$ alkyl;
X is Br, Cl or I; and
W, L, M, and R are as defined in claim 1, which comprises reacting a compound of formula V chosen from compounds of claim 1

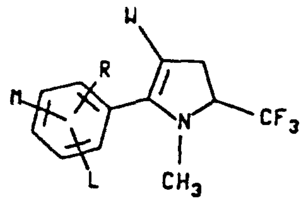

(V)

wherein W, L, M and R are as described herein above with at least 1 equivalent of a halogen, $X_2$, wherein X is Br, Cl or I, in the presence of a solvent to obtain a 2-aryl-1-methylpyrrole intermediate, reacting said 1-methylpyrrole intermediate with at least one additional molar equivalent of said halogen to form a 2-aryl-4-halo-1-methylpyrrole intermediate, reacting said 4-halo-1-methylpyrrole intermediate further with at least one molar equivalent of said halogen in the presence of a radical initiator to form a 2-aryl-4-halo-1-(halomethyl)pyrrole intermediate and reacting said 4-halo-1-(halomethyl)pyrrole intermediate with at least one molar equivalent of an alkali metal $C_1$-$C_6$-alkoxide to give the compound of formula IV.

## Patentansprüche

**1.** Verbindung **gekennzeichnet durch** die Struktur I

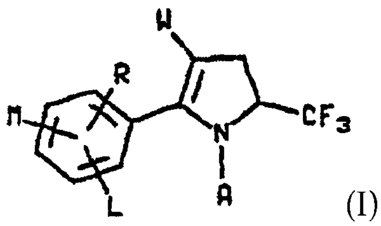

(I)

,

in der

A Wasserstoff oder $C_1$-$C_4$Alkyl bedeutet,
W CN, $NO_2$ oder $CO_2R_6$ bedeutet,
L Wasserstoff oder Halogen bedeutet und
M und R jeweils unabhängig voneinander Wasserstoff, $C_1$-$C_4$Alkyl, $C_1$-$C_4$Alkoxy, $C_1$-$C_4$Alkylthio, $C_1$-$C_4$Alkylsulfinyl, $C_1$-$C_4$Alkylsulfonyl, CN, $NO_2$, Halogen, $CF_3$, $R_1CF_2Z_1R_2CO$ oder $NR_3R_4$ bedeuten und, wenn sich M und R in benachbarten Positionen befinden, sie gemeinsam mit den Kohlenstoffatomen, an die sie gebunden sind, einen Ring bilden können, in dem MR die Struktur

$$-OCH_2O-, -OCF_2O- \text{ oder } -CH=CH-CH=CH-$$

bedeutet,

Z $S(O)_n$ oder O bedeutet,
$R_1$ Wasserstoff, F, $CHF_2$, CHFCl oder $CF_3$ bedeutet,
$R_2$ $C_1$-$C_4$Alkyl, $C_1$-$C_4$Alkoxy oder $NR_3R_4$ bedeutet,
$R_3$ Wasserstoff oder $C_1$-$C_4$Alkyl bedeutet,
$R_4$ Wasserstoff, $C_1$-$C_4$Alkyl oder $R_5CO$ bedeutet,
$R_5$ Wasserstoff oder $C_1$-$C_4$Alkyl bedeutet und
$R_6$ $C_1$-$C_6$Alkyl, $C_3$-$C_6$Cycloalkyl oder Phenyl bedeutet,
n eine ganze Zahl 0, 1 oder 2 bedeutet.

2. Verbindung nach Anspruch 1, aus der Gruppe 2-(p-Chlorphenyl)-1-methyl-5-trifluormethyl)-2-pyrrolin-3-carbonsäurenitril und 2-(p-Chlorphenyl)-5-(trifluormethyl)-2-pyrrolin-3-carbonsäurenitril.

3. Verfahren zur Herstellung einer Verbindung nach Anspruch 1, **dadurch gekennzeichnet, daß** man eine Verbindung der Struktur II

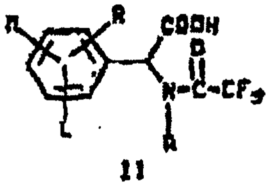

II

,

in der A, L, M und R wie in Anspruch 1 definiert sind, mit ungefähr 1,0 Moläquivalent eines Olefins der Struktur

$$H_2C=CHW,$$

in der W wie oben definiert ist, sowie gegebenenfalls einer katalytischen Menge einer organischen Base in Gegenwart eines Säureanhydrids und eines Lösungsmittels umsetzt.

4. Verfahren nach Anspruch 3, bei dem die Verbindung der Struktur II

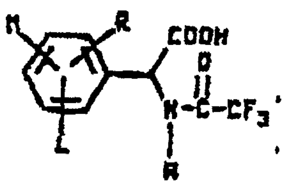

II

,

in der A, L, M und R wie in Anspruch 1 definiert sind, unter der Voraussetzung, daß, wenn A Wasserstoff bedeutet, mindestens einer der Substituenten L, M, und R nicht Wasserstoff sein darf, dadurch hergestellt wird, daß man einen Aldehyd mit der Struktur

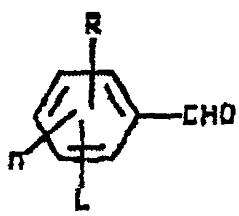

,

in der L, M und R wie oben definiert sind, mit mindestens einem Moläquivalent eines Aminhydrochlorids der Struktur

$$H_2N\text{-}A \cdot HCl,$$

in der A wie oben beschrieben, in Gegenwart eines Lösungsmittels umsetzt, wodurch man zu einem Zwischenprodukt gelangt, diese Zwischenproduktlösung mit einer wäßrigen Lösung, die mindestens ein Moläquivalent eines Alkalimetallcyanids enthält, umsetzt, wodurch man zu einem Aminonitrilzwischenprodukt gelangt, dieses Aminonitrilzwischenprodukt mit einem geeigneten Lösungsmittel aus der Reaktionsmischung extrahiert, das Aminonitrilzwischenprodukt mit einem Säureanhydrid in Gegenwart einer organischen Base umsetzt, wodurch man zu einem Amidonitrilzwischenprodukt gelangt, das Amidonitril mit einer Mineralsäure in Gegenwart von Wasser sowie gegebenenfalls bei erhöhter Temperatur umsetzt, wodurch man zu einem Aminosäurezwischenprodukt gelangt, und dieses Zwischenprodukt mit Trifluoressigsäureanhydrid umsetzt, wodurch man zu der erwünschten Verbindung der Struktur II gelangt.

**5.** Verfahren zur Herstellung einer der Formel IV

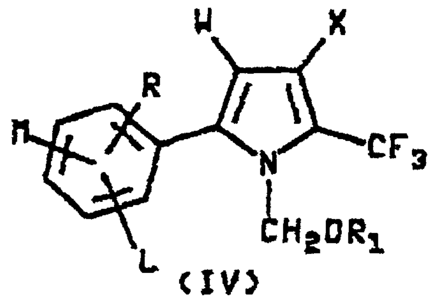

(IV)

entsprechenden Verbindung, in der

$R_1$ $C_1$-$C_6$Alkyl bedeutet,
X Br, Cl oder I bedeutet, und
W, L, M und R wie in Anspruch 1 definiert sind, **dadurch gekennzeichnet, daß** man eine Verbindung der Formel V aus der Gruppe der Verbindungen von Anspruch 1

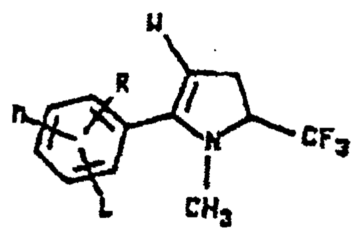

(V)

,

in der W, L, M und R wie im vorliegenden Text beschrieben sind, mit mindestens 1 Äquivalent eines Halogens $X_2$, wobei X Br, Cl oder I bedeutet, in Gegenwart eines Lösungsmittels umsetzt, wodurch man zu einem 2-Aryl-1-methylpyrrolzwischenprodukt gelangt, dieses 1-Methylpyrrolzwischenprodukt mit mindestens einem weiteren Moläquivalent des Halogens umsetzt, wodurch man zu einem 2-Aryl-4-halogen-1-methylpyrrolzwischenprodukt gelangt, dieses 4-Halogen-1-methylpyrrolzwischenprodukt weiter mit mindestens einem Moläquivalent des Halogens in Gegenwart eines Radikalbildners umsetzt, wodurch man zu einem 2-Aryl-4-halogen-1-(halogenmethyl)pyrrolzwischenprodukt gelangt, und dieses 4-Halogen-1-(halogenmethyl)pyrrolzwischenprodukt mit mindestens einem Moläquivalent eines Alkalimetall-$C_1$-$C_6$alkoholats umsetzt, wodurch man zu der Verbindung der Formel IV gelangt.

## Revendications

**1.** Composé **caractérisé par** la structure I

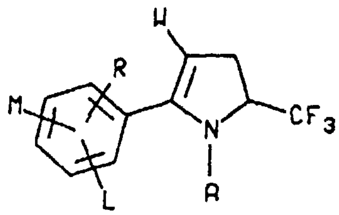

I

où

A est l'hydrogène ou un alkyle en $C_1$-$C_4$;
W est CN, $NO_2$ ou $CO_2R_6$;
L est l'hydrogène ou un halogène; et
M et R sont chacun indépendamment l'hydrogène, un alkyle en $C_1$-$C_4$, un alcoxy en $C_1$-$C_4$, un alkylthio en $C_1$-$C_4$, un alkylsulfinyle en $C_1$-$C_4$, un alkylsulfonyle en $C_1$-$C_4$, CN, $NO_2$, un halogène, $CF_3$, $R_1CF_2Z$, $R_2CO$ ou $NR_3R_4$ et, lorsque M et R sont sur des positions adjacentes, ils peuvent être pris conjointement avec les atomes de carbone auxquels ils sont liés pour former un cycle dans lequel MR représente la structure

$$-OCH_2O- \ -OCF_2O- \text{ o } -CH=CH-CH=CH- ;$$

Z est $S(O)_n$ ou O;
$R_1$ est l'hydrogène, F, $CHF_2$, CHFCl ou $CF_3$;
$R_2$ est un alkyle en $C_1$-$C_4$, un alcoxy en $C_1$-$C_4$ ou $NR_3R_4$;
$R_3$ est l'hydrogène ou un alkyle en $C_1$-$C_4$;
$R_4$ est l'hydrogène, un alkyle en $C_1$-$C_4$ ou $R_5CO$;
$R_5$ est l'hydrogène ou un alkyle en $C_1$-$C_4$ et
$R_6$ est un alkyle en $C_1$-$C_6$, un cycloalkyle en $C_3$-$C_6$ ou un phényle;
n est un entier valant 0, 1 ou 2.

2. Composé selon la revendication 1, choisi dans le groupe consistant en le 2-(p-chlorophényl)-1-méthyl-5-trifluorométhyl)-2-pyrroline-3-carbonitrile et le 2-(p-chlorophényl)-5-(trifluorométhyl)-2-pyrroline-3-carbonitrile.

3. Procédé pour la préparation d'un composé selon la revendication 1 qui comprend la réaction d'un composé ayant la structure II

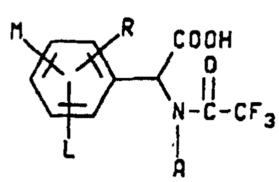

où A, L, M et R sont tels que définis dans la revendication 1, avec environ 1,0 équivalent molaire d'une oléfine ayant la structure

$$H_2C=CHW,$$

où W est tel que défini plus haut et en option une quantité catalytique d'une base organique en présence d'un anhydride d'acide et d'un solvant.

4. Procédé selon la revendication 3, selon lequel le composé de structure II

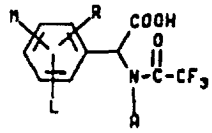

II

où A, L, M et R sont tels que définis dans la revendication 1, avec pour condition de quand A est l'hydrogène alors au moins l'un des L, M et R doit être autre que l'hydrogène, est préparé par réaction d'un aldéhyde ayant la structure

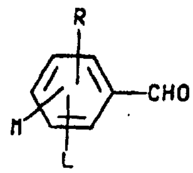

où L, M et R sont tels que définis plus haut, avec au moins un équivalent molaire d'un chlorhydrate d'amine ayant la structure

$$H_2N\text{-}A\cdot HCl$$

où A est tel que décrit plus haut, en présence d'un solvant pour former un intermédiaire, réaction de ladite solution intermédiaire avec une solution aqueuse contenant au moins un équivalent molaire d'un cyanure de métal alcalin pour former un intermédiaire amino nitrile, l'extraction du dit intermédiaire amino nitrile du mélange réactionnel avec un solvant approprié, la réaction de l'intermédiaire amino nitrile avec un anhydride d'acide en présence d'une base organique pour former un intermédiaire amido nitrile, la réaction du dit amido nitrile avec un acide minéral en présence d'eau, en option à une température élevée, pour donner un intermédiaire amino acide, et la réaction du dit intermédiaire avec l'anhydride trifluoroacétique pour donner le composé souhaité ayant la structure II.

**5.** Procédé pour la préparation d'un composé **caractérisé par** la formule IV

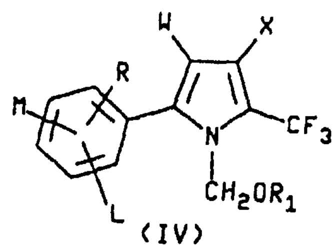

(IV)

où

$R_1$ est un alkyle en $C_1$-$C_6$;
X est Br, Cl ou I; et
W, L, M et R sont tels que définis dans la revendication 1, qui comprend la réaction d'un composé de formule V choisi parmi les composés selon la revendication 1

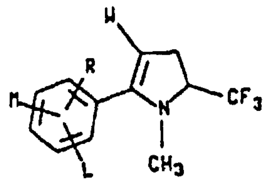

(V)

où W, L, M et R sont tels que décrits ci-dessus avec au moins 1 équivalent d'un halogène, $X_2$, où X est Br, Cl ou I, en présence d'un solvant pour obtenir un intermédiaire 2-aryl-1-méthylpyrrole, la réaction du dit intermédiaire 1-méthylpyrrole avec au moins un équivalent molaire additionnel du dit halogène pour former un intermédiaire 2-aryl-4-halogéno-1-méthylpyrrole, la réaction du dit intermédiaire 4-halogéno-1-méthylpyrrole encore avec au moins un équivalent molaire du dit halogène en présence d'un initiateur radicalaire pour former un intermédiaire 2-aryl-4-halogéno-1-(halogénométhyl)pyrrole et la réaction du dit intermédiaire 4-halogéno-1-(halogénométhyl) pyrrole avec au moins un équivalent molaire d'un alcoolate en $C_1$-$C_8$ de métal alcalin pour donner le composé de formule IV.